# EUROPEAN PATENT APPLICATION

(11) **EP 1 321 766 A1**
(43) Date of publication of application: **25.06.2003**
(21) Application number: 02027325.6
(22) Date of filing: 06.12.2002
(51) Int. Cl.: G01N 27/62, G01N 27/68, G01N 33/00, G01N 33/497

(54) **Mass spectrometer for detecting stowaways in containers**

(30) Priority: 19.12.2001 JP 2001385378
(71) Applicant: Hitachi, Ltd., Chiyoda-ku, Tokyo 101-8010 (JP)
(72) Inventor: Nakashige, Keiko, Hitachi-shi, Ibaraki 316-0035 (JP); Tsutsumi, Hirofumi, Tokyo 154-0002 (JP); Tanaka, Seiji, Hitchinaka-shi, Ibaraki 312-0003 (JP); Takada, Yasuaki, Kiyose-shi, Tokyo 204-0023 (JP)
(74) Representative: Strehl Schübel-Hopf & Partner

(57) **Abstract**

An apparatus and a method for inspecting the inside of a container (1) can easily detect a stowaway being in hiding in the container (1). The apparatus includes a sampling probe (6) adapted to be disposed at an appropriate position inside the container (1) for sampling an atmospheric gas inside the container (1), a mass spectrometer (4) for detecting the atmospheric gas sampled by the probe (6) as a molecular weight by ionizing the atmospheric gas, and a control processing part (5) for comparing the information detected by the mass spectrometer (4) with a pre-registered index molecular weight to judge whether or not an abnormality exists.

## Description

### BACKGROUND OF THE INVENTION

The present invention relates to an apparatus for inspecting the inside of a container and a method of inspecting the inside of a container, and particularly to an apparatus for inspecting the inside of a container and a method of inspecting the inside of a container which are suitable for detecting stowaways inside a container at a public facility such as an airport or a harbor.

An apparatus using X-rays has been known as a conventional apparatus for inspecting the inside of a container. This is an apparatus which performs a non-destructive inspection (a transmission inspection or a tomography inspection) of a container using X-rays in order to disclose social evil articles or undeclared articles concealed inside the container using information obtained through an image analysis.

On the other hand, an apparatus for detecting an explosive using a mass spectrometer is disclosed in Japanese Patent Application Laid-Open No.2000-28579. This is an apparatus which detects an explosive by ionizing a sample gas, and is expected to be used at an airport or the like.

The conventional technology described above is for detecting social evil articles or undeclared articles concealed inside the container from the information obtained through an image analysis using X-rays. However, in recent years, number of stowaways being in hiding in a container is being increased, and accordingly there is the following problem when the conventional technology using X-rays is applied to the detection of the stowaways. That is, although the inspection apparatus using X-rays can check whether or not there is something inside the container, it is difficult to judge which the thing is, a stowaway, a social evil article or an undeclared article. Further, the inspection apparatus using X-rays may adversely affect the stowaway (a human being) because of using the X-rays.

On the other hand, the apparatus for detecting an explosive using the analyzer, the object to be detected is an explosive. Accordingly, the gases to be detected are completely different from kinds of the detected gases for detecting the stowaway. Furthermore, in the case of the stowaway, since the baggage carried by the stowaway should be passed through an inspection apparatus, the inspection apparatus is unsuitable for inspecting the inside of the large-sized container.

### SUMMARY OF THE INVENTION

The present invention is made in order to solve the above problems. A first object of the present invention is to provide an apparatus for inspecting the inside of a container which can easily detect a stowaway being in hiding in the container, and to provide a method of inspecting the inside of the container. In addition to the first object, a second object of the present invention is to provide an apparatus for inspecting the inside of a container which can also detect social evil articles or undeclared articles.

In order to attain the first object described above, an apparatus for inspecting an inside of a container is characterized by that the apparatus comprises a sampling probe for sampling an atmospheric gas inside the container, the sampling probe being arranged at an appropriate position inside the container; a mass spectrometer for detecting the atmospheric gas sampled by the sampling probe as a molecular weight by ionizing the atmospheric gas; and a control processing part for comparing the information detected by the mass spectrometer with a pre-registered index molecular weight to judge whether or not an abnormality exists.

Further, in order to attain the second object described above, in addition to the inspection apparatus described above, the present invention is characterized by that an apparatus for inspecting an inside of a container comprises an X-ray CT inspection system for further inspecting the inside of the container after judgment by the control processing part, and in addition to the inspection method described above, the present invention is characterized by that a method of inspecting the inside of a container further comprises the step of inspecting the container using an X-ray CT system.

Further, the present invention is characterized by that an apparatus for inspecting an inside of a container further comprises a heating heater in a gas introducing pipe of the sample probe.

Further, the present invention is characterized by that a method of inspecting the inside of a container comprises the steps of sampling an atmospheric gas filled in the container and ionizing the sampled atmospheric gas at a discharge part of a mass spectrometer; at the same time detecting the ionized atmospheric gas as a molecular weight; and performing a detailed inspection of the inside of the container when the molecular weight corresponds to a pre-registered index molecular weight of human smell.

Further, in addition to the inspection method described above, the present invention is characterized by that a method of inspecting the inside of a container further comprises the step of performing inspection of the inside of the container using an X-ray CT system.

### BRIEF DESCRIPTION OF THE DRAWINGS

The present invention will be understood more fully from the detailed description given hereinafter and from the accompanying drawings of the preferred embodiment of the present invention, which, however, should not be taken to be limitative to the invention, but are for explanation and understanding only.

In the drawings:
FIG. 1 is a view showing the construction of an apparatus for inspecting gases inside a container in accordance with the present invention.
FIG. 2 is a view showing the construction in a case of combining the apparatus for inspecting gases inside a container in accordance with the present invention with an X-ray equipment.
FIG. 3 is a view showing the construction of a sampling probe in accordance with the present invention.
FIG. 4 is a flowchart showing the detection procedure in accordance with the present invention.
Fig. 5 is an illustration showing a construction of a container inspection apparatus.

### DESCRIPTION OF THE PREFERRED EMBODIMENT

The present invention will be discussed hereinafter in detail in terms of the preferred embodiment of the present invention with reference to the accompanying drawings. In the following description, numerous specific details are set forth in order to provide a thorough understanding of the present invention. It will be obvious, however, to those skilled in the art that the present invention may be practiced without these specific details. In other instance, well-known structures are not shown in detail in order to avoid unnecessary obscurity of the present invention. An apparatus for inspecting the inside of a container in accordance with the present invention will be described below, referring to an illustrated embodiment.

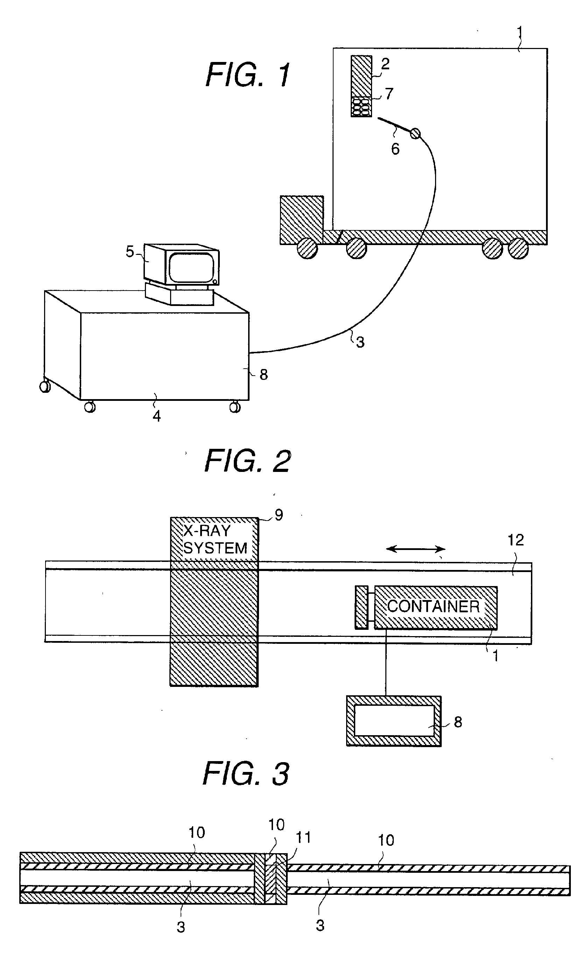

FIG. 1 is a schematic view showing the construction of an embodiment of an apparatus for inspecting gases inside a container in accordance with the present invention. A container 1 in the present embodiment is a container which has been transported to an airport or a harbor from a foreign country, and is carried into an inspection facility (for example, a custom house in the airport or the harbor) from there by a truck or the like to be inspected. A vent port 2 composed of a plurality of vent port holes 7 is provided at an appropriate position of the container 1. (The vent port 2 is arranged generally at a corner the container 1 because the container 1 is a large freight.)

An apparatus 8 for inspecting the inside of the container of the present embodiment comprises a sampling probe 6 for sampling an atmospheric gas emitted from a stowaway inside the container 1 by being inserted into a vent port hole 7 of the container; a mass spectrometer 4 for detecting the atmospheric gas sampled by the sampling probe 6 as a molecular weight by ionizing the atmospheric gas at its discharging part; a gas sucking pipe 3 for transporting the atmospheric gas inside the container 1 sampled by the sampling probe 6 to the mass spectrometer 4 by connecting the mass spectrometer 4 with the sampling probe 6; and a control processing part 5 for judging whether or not there is an abnormality by comparing the information (human smell of a stowaway) detected by the above-described mass spectrometer 4 with a pre-registered index molecular weight of human smell.

An inspector inserts the sampling probe 6 attached to a top end of the gas sucking pipe 3 into the vent port hole 7 to sucking and introduce the atmospheric gas inside the container 1 into the mass spectrometer 4. It is preferable that the structure of the top end of the sampling probe 6 is so slender as to be easily inserted into the vent port hole 7.

Further, as shown in FIG. 3, a heating heater 10 is arranged in the gas sucking pipe 3 for transporting the atmospheric gas inside the container 1 to the mass spectrometer 4, and the heating heater 10 removes water droplets produced by rain water, sea water or the like attached on the surface of the container 1 and prevents the sucked gas inside the container 1 from being adsorbed to the inner surface of the gas sucking pipe 3.

Further, when the apparatus is used at apposition near a harbor, it is advantageous that an anti-salt-damage filter 11 is arranged to the gas sucking pipe 3. Furthermore, by forming the filter 11 detachable, dust attached to the filter 11 can be easily removed.

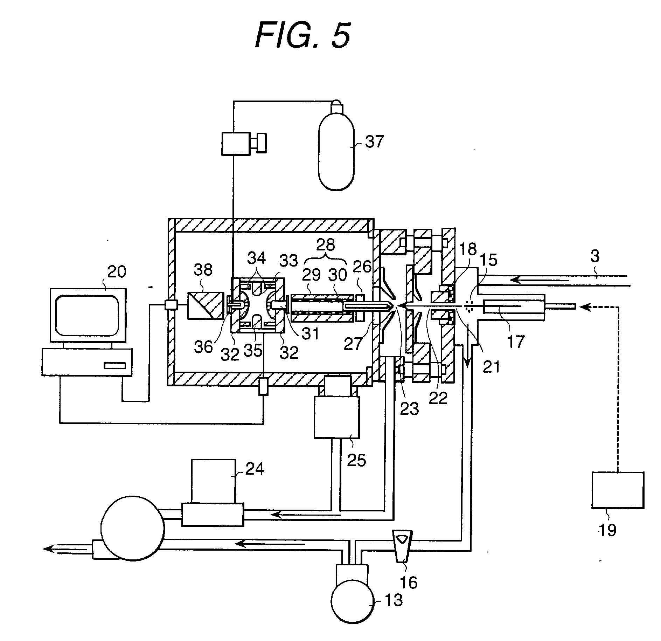

Fig. 5 shows a particular construction of a container inspection apparatus for implementing the present invention. In the embodiment shown in Fig. 5, a quadrupole ion trap mass spectrometer (hereinafter referred to as ion trap mass spectrometer) is employed in a mass analyzing portion. A gas sample is sucked by the gas suction pump 13 and is introduced into the corona discharge portion 15 for ionizing through the gas suction pipe 3. A suction force of the gas suction pump 13 can be adjusted by a flow meter 16. The gas sample thus introduced is fed into a corona discharge region at the tip end of a needle electrode 17 for efficient corona discharge. In the corona discharge portion 15, the corona discharge needle electrode 17 and a drawer electrode 18 as a counter electrode are provided for applying a high voltage by a corona discharge region 19. The fed gas sample is ionized by corona discharge and is detected as molar weight in the analyzing portion. The molar weight is preliminarily input to a data processing portion 20 to compare with the analyzed molar weight. If the analyzed molar weight matches with the molar weight of the registered index material, further detailed inspection is performed. Ion generated by an ion source and gas introduced into the ion source is taken into a vacuum portion ventilated by a vacuum pump 24 through a first aperture 21, a middle aperture 22 and a second aperture 23. Diameters of these apertures are about 0.3 mm. Electrodes, in which the apertures are open, are heated by a heater.

Differential pumping regions are defined between electrodes, to which the apertures are open for ventilation by roughing vacuum pump. As the roughing vacuum pump, a rotary pump, a scroll pump, a mechanical booster pump and so forth may be employed normally. It is also possible to use a turbo-molecular pump 25 for ventilation in this region. On the other hand, voltage is applied to the electrodes, to which the apertures are open, for improvement of ion transmittivity, and in conjunction therewith to adjust cluster ion generated by adiabatic expansion due to collision with residual molecule.

After passing through the second aperture 23, the generated ion is converged by a convergence lens 26. As convergence lens, Einzel lens normally consisted of three electrodes and so forth is used. Ion also passes through a slit electrode 27. Ion passed through the second aperture converges to an opening portion of the slit electrode 27 by the convergence lens 26 to pass therethrough. Neutral particle or the like which is not converged collides on the slit portion to hardly reach to the mass analyzing portion. Ion passed through the electrode with the slit is deflected and converged by a double cylinder type deflector 28 consisted of an inside pipe electrode 29 and an outside pipe electrode 30 having large number of opening portions. In the double cylinder type deflector 28, the electric field of the outside pipe electrode 30 exuded from the opening portion of the inside pipe electrode 29 is used for deflection and convergence of ion.

Ion passed through the double cylinder deflector 28 is introduced into the ion trap mass spectrometer. Ion trap is constructed with a gate electrode 31, an endcap electrode 32, a ring electrode, a guard electrode 34, an insulation ring 35 and a ion taking-out lens 36. The gate electrode 31 servers for blocking introduction of ion into the ion trap mass spectrometer upon taking out ion captured in the ion trap mass analyzing portion. Ion introduced into the ion trap mass analyzing portion through an ion taking-out conduit is discharged outside of the ion trap mass analyzing portion through the ion taking-out aperture per mass number by scanning a high frequency voltage applied between the endcap electrode 32 and the ring electrode 33 and detected by a detector 38, after narrowing of path by collision with a buffer gas 37, such as helium, introduced within the ion trap mass analyzing portion.

A method of inspecting the inside of the container will be described below. Initially, the atmospheric gas sampled from the inside of the container 1 is ionized in the mass spectrometer 4 using corona discharge, and then the ionized atmospheric gas is detected as a molecular weight. This information is transmitted to the control processing part 5, and it is judged whether or not the detected molecular weight corresponds to a pr-registered index molecular weight of human smell, and then the detected result is displayed on a display part. For example, if an index substance A is detected (if there is a stowaway), the detection of the substance A (the detection of the stowaway) is indicated by flashing a lamp A. At that time, a quantity or an alarm indicating what amount of concentration is detected may be displayed at the same time.

By aiming at substances contained in breath and excrement peculiar to human beings such as ammonia, acetone etc as the index objects for detecting a stowaway, a human being inside the container can be detected by detecting these substances. Particularly, ammonia has a high ionization efficiency and accordingly is suitable as the index. However, there is possibility that the detection of only ammonia can not discriminate between a human being and a rat or the like. In such a case, human smell is judged based on a theory of judging human smell by combining the other index substances. As the other index substances, there are substances essential to perspiration such as a substance having smell specific to perspiration (iso-varelic acid), substances causing bad smell such as lower fatty acid (caproic acid) amines, aldehydes etc.

FIG. 2 is a schematic view showing an in-container inspection apparatus which is combined with an X-ray inspection system 9.

As shown in this figure, the container 1 is mounted on a belt conveyer 12, and the atmospheric gas inside the container 1 is inspected using the in-container inspection apparatus 8, as described above, in order to judge whether or not there is any stowaway inside the container. After checking that there is no stowaway in the container, the container 1 is transported to the X-ray inspection system 9 by the conveyer 12 to inspect whether or not there is any suspected article other than declared articles.

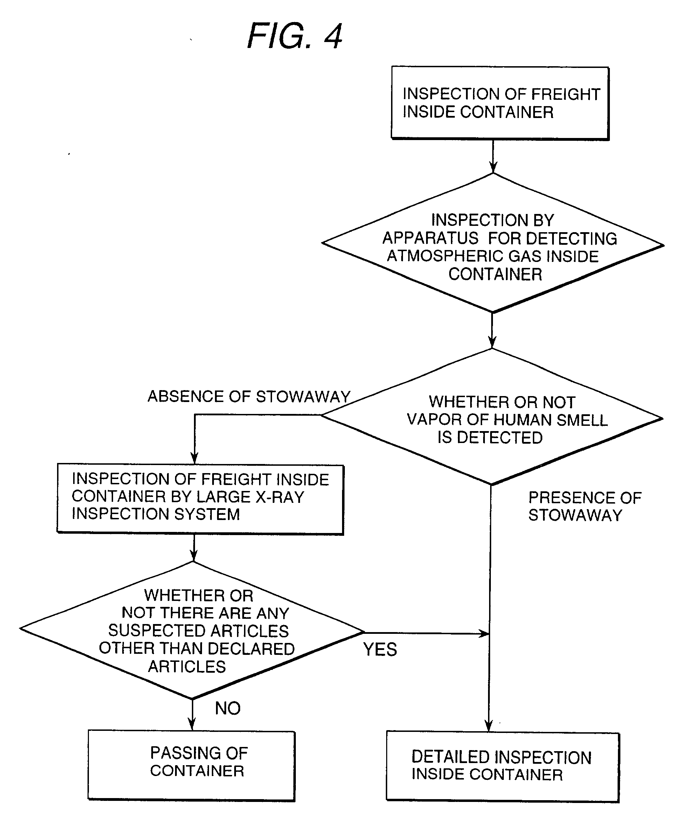

The detailed explanation of the operation will be described below, referring to the inspection flow of FIG. 4.

When the inspection inside the container is performed, the atmospherics gas inside the container is initially detected using the sampling probe and the mass spectrometer.

Next, when vapor of human smell is detected in the detected gas, an alarm indicating occurrence of an abnormality is produced by flashing of a lamp or sounding of alarm, and at the same time a detailed inspection inside the container is made.

As a method for enhancing selectivity in mass spectrometer, a tandem mass analyzing method (hereinafter referred to as MS/MS) has been known. As an apparatus for implementing MS/MS, triple quadrupole mass spectrometer, quadrupole mass spectrometer and so forth may be used. Even if m/z of ion generated by ion source is accidentally the same, whether objective substance to be detected is contained or not by checking mass spectrum of fragment ion. Accordingly, by performing MS/MS, selectivity can be improved and erroneous detection can be reduced.

In order to further enhance selectivity, further higher order mass analysis may be performed. Namely, it may be possible to provide a step of selecting ion having particular m/z among fragment ion and performing mass analysis of ion obtained by dissociation of the ion interested (this will be referred to as MS/MS/MS or MS³). Process of such selection, dissociation and mass analysis may be repeated until desired selectivity is satisfied.

On the other hand, when vapor of human smell is not detected in the detected gas, a non-destructive inspection (for example, a transparent inspection and a tomography inspection) of the inside of the container is performed using X-rays.

When no abnormality is observed and when there is no suspected article other than declared articles as the non-destructive inspection using X-rays, the container is passed as it is.

On the other hand, when any abnormality is observed as the non-destructive inspection using X-rays, a detailed inspection inside the container is performed.

By performing the inspections described above, it is possible to easily detect a stowaway when the stowaway is in hiding inside the container before performing the X-ray inspection, and accordingly it is possible to prevent the stowaway from entering into a country.

Furthermore, by turning ON corona discharge in the ion source portion only upon performing inspection of the inspection object, data with high precision, reproductivity and reliability can be obtained even when long continuous operation is performed.

It is possible to provide a ON/OFF switch for corona discharge in a grip portion of a sampling probe 6 for container analysis sucking gas within the container. By inserting the sampling probe into the ventilation hole and turning ON the ON/OFF switch for corona discharge, a power source 19 for corona discharge becomes ON. Thus, gas sample is ionized by corona discharge. Ionized ion is analyzed in the analyzing portion. When container inspection is not performed, corona discharge is not effected in the ion source portion 15, deposition of Si, C or the like within the ion source including the needle electrode 17 and drawer electrode 18 can be reduced. When the container inspection is not performed, the gas sample is not introduced into the inside of the ion source. This is particularly effective for large cargo, such as cargo container since gas sample therein may contain salt or water vapor. Furthermore, since the ON/OFF switch is located in the grip portion of the sampling probe, the operator can easily operate.

In a public facility such as an airport, a harbor or the like, an apparatus for inspecting a large-sized freight such as the container or the like is sometimes installed outdoors. Therefore, the apparatus for inspecting the inside of the container needs to be heat resistant, water proof and salt-damage resistant. In such a case, the apparatus for inspecting the inside of the container can be protected the apparatus from the effects of salt damage, rain water and so on by installing the apparatus inside a cubicle.

According to the present invention described above, there are an effect that a stowaway being in hiding in the container can be detected and also an effect that social evil articles or undeclared articles can be detected. Therefore, the present invention is very effective for inspection at an airport and a harbor.

Although the present invention has been illustrated and described with respect to exemplary embodiment thereof, it should be understood by those skilled in the art that the foregoing and various other changes, omission and additions may be made therein and thereto, without departing from the spirit and scope of the present invention. Therefore, the present invention should not be understood as limited to the specific embodiment set out above but to include all possible embodiments which can be embodied within a scope encompassed and equivalent thereof with respect to the feature set out in the appended claims.

## Claims

1. An apparatus for inspecting the inside of a container (1), comprising
a sampling probe (6) adapted to be placed at an appropriate position inside the container (1) for sampling an atmospheric gas inside the container (1),
a mass spectrometer (4) for detecting the atmospheric gas sampled by said probe (6) as a molecular weight by ionizing said atmospheric gas; and
a control processing part (5) for comparing the information detected by said mass spectrometer (4) with a pre-registered index molecular weight to judge whether or not an abnormality exists.

2. The apparatus of claim 1, comprising an X-ray CT inspection system (9) for further inspecting the inside of the container (1) after judgment by said control processing part (5).

3. The apparatus of claim 1 or 2, wherein said probe (6) and said mass spectrometer (4) are interconnected by a gas introducing pipe (3) in which a heater (10) is provided.

4. A method of inspecting the inside of a container (1), comprising
sampling an atmospheric gas inside the container (1) and ionizing the sampled atmospheric gas at a discharge part of a mass spectrometer (4);
at the same time detecting the ionized atmospheric gas as a molecular weight; and
performing a detailed inspection of the inside of the container (1) when said molecular weight corresponds to a pre-registered index molecular weight of human smell.

5. The method of claim 4 further comprising performing inspection of the inside of the container (1) using an X-ray CT system (9).
